# EUROPEAN PATENT SPECIFICATION

(11) **EP 2 097 511 B1**
(45) Date of publication and mention of the grant of the patent: **15.09.2010**
(21) Application number: 07866642.7
(22) Date of filing: 30.11.2007
(51) Int. Cl.: C12N 1/18, C12N 1/19, C12P 11/00, C12G 1/00

(54) **USE OF URE2 MUTANT YEASTS FOR INCREASING THE RELEASE OF AROMATIC VOLATILE THIOLS BY YEAST DURING FERMENTATION**
VERWENDUNG VON MUTANTEN URE2-HEFEN ZUR ERHÖHUNG DER FREISETZUNG VON FLÜCHTIGEN AROMATISCHEN THIOLEN DURCH HEFE WÄHREND DER GÄRUNG
UTILISATION DE LEVURES MUTANTES URE2 POUR AUGMENTER LA LIBERATION DE THIOLS VOLATILS AROMATIQUES DE LA LEVURE LORS DE LA FERMENTATION

(30) Priority: 01.12.2006 EP 06291855
(43) Date of publication of application: 09.09.2009
(73) Proprietor: Sarco, 33015 Bordeaux Cedex (FR); Université Victor Segalen Bordeaux 2, 33076 Bordeaux Cedex (FR)
(72) Inventor: MARULLO, Philippe, 33370 Tresses (FR); THIBON, Cecile, 33800 Bordeaux (FR); DUBOURDIEU, Denis, 33410 Beguey (FR); TOMINAGA, Takatoshi, 33000 Bordeaux (FR)
(74) Representative: Leblois-Préhaud, Hélène Marthe Georgette
(86) International application number: PCT/IB2007/004383
(87) International publication number: WO 2008/068635

(56) References cited:
- SALMON JEAN-MICHEL ET AL: "Improvement of nitrogen assimilation and fermentation kinetics under enological conditions by depression of alternative nitrogen-assimilatory pathways in an industrial Saccharomyces cerevisiae strain" APPLIED AND ENVIRONMENTAL MICROBIOLOGY, vol. 64, no. 10, October 1998 (1998-10), pages 3831-3837, XP002428301 ISSN: 0099-2240 cited in the application
- HOWELL KATE S ET AL: "Genetic determinants of volatile-thiol release by Saccharomyces cerevisiae during wine fermentation" APPLIED AND ENVIRONMENTAL MICROBIOLOGY, vol. 71, no. 9, September 2005 (2005-09), pages 5420-5426, XP002428302 ISSN: 0099-2240 cited in the application
- PRETORIUS ISAK S: "Tailoring wine yeast for the new millennium: Novel approaches to the ancient art of winemaking" YEAST, vol. 16, no. 8, 15 June 2000 (2000-06-15), pages 675-729, XP002428303 ISSN: 0749-503X

### SEQUENCE LISTING

<110> SARCO
   UNIVERSITE VICTOR SEGALEN BORDEAUX 2
   INSTITUT NATIONAL DE LA RECHERCHE AGRONOMIQUE
   MARULLO, Philippe
   THIBON, Cécile
   DUBOURDIEU, Denis
   TOMINAGA, Takatoshi
<120> Use of URE2 mutant yeasts for increasing the release of aromatic volatile thiols by yeast during fermentation
<130> s2012PCT1
<160> 17
<170> PatentIn version 3.3
<210> 1
   <211> 354
   <212> PRT
   <213> Saccharomyces cerevisiae
<400> 1
   Met Met Asn Asn Asn Gly Asn Gln Val Ser Asn Leu Ser Asn Ala Leu
   1               5                   10                  15
   Arg Gln Val Asn Ile Gly Asn Arg Asn Ser Asn Thr Thr Thr Asp Gln
               20                  25                  30
   Ser Asn Ile Asn Phe Glu Phe Ser Thr Gly Val Asn Asn Asn Asn Asn
           35                  40                  45
   Asn Asn Ser Ser Ser Asn Asn Asn Asn Val Gln Asn Asn Asn Ser Gly
       50                  55                  60
   Arg Asn Gly Ser Gln Asn Asn Asp Asn Glu Asn Asn Ile Lys Asn Thr
   65                  70                  75                  80
   Leu Glu Gln His Arg Gln Gln Gln Gln Ala Phe Ser Asp Met Ser His
                   85                  90                  95
   Val Glu Tyr Ser Arg Ile Thr Lys Phe Phe Gln Glu Gln Pro Leu Glu
               100                 105                 110
   Gly Tyr Thr Leu Phe Ser His Arg Ser Ala Pro Asn Gly Phe Lys Val
           115                 120                 125
   Ala Ile Val Leu Ser Glu Leu Gly Phe His Tyr Asn Thr Ile Phe Leu
       130                 135                 140
   Asp Phe Asn Leu Gly Glu His Arg Ala Pro Glu Phe Val Ser Val Asn
   145                 150                 155                 160
   Pro Asn Ala Arg Val Pro Ala Leu Ile Asp His Gly Met Asp Asn Leu
                   165                 170                 175
   Ser Ile Trp Glu Ser Gly Ala Ile Leu Leu His Leu Val Asn Lys Tyr
               180                 185                 190
   Tyr Lys Glu Thr Gly Asn Pro Leu Leu Trp Ser Asp Asp Leu Ala Asp
           195                 200                 205
   Gln Ser Gln Ile Asn Ala Trp Leu Phe Phe Gln Thr Ser Gly His Ala
       210                 215                 220
   Pro Met Ile Gly Gln Ala Leu His Phe Arg Tyr Phe His Ser Gln Lys
   225                 230                 235                 240
   Ile Ala Ser Ala Val Glu Arg Tyr Thr Asp Glu Val Arg Arg Val Tyr
                   245                 250                 255
   Gly Val Val Glu Met Ala Leu Ala Glu Arg Arg Glu Ala Leu Val Met
               260                 265                 270
   Glu Leu Asp Thr Glu Asn Ala Ala Ala Tyr Ser Ala Gly Thr Thr Pro
           275                 280                 285
   Met Ser Gln Ser Arg Phe Phe Asp Tyr Pro Val Trp Leu Val Gly Asp
       290                 295                 300
   Lys Leu Thr Ile Ala Asp Leu Ala Phe Val Pro Trp Asn Asn Val Val
   305                 310                 315                 320
   Asp Arg Ile Gly Ile Asn Ile Lys Ile Glu Phe Pro Glu Val Tyr Lys
                   325                 330                 335
   Trp Thr Lys His Met Met Arg Arg Pro Ala Val Ile Lys Ala Leu Arg
               340                 345                 350
   Gly Glu
<210> 2
   <211> 22
   <212> DNA
   <213> artificial sequence
<220>
   <223> primer
<400> 2 caaatgccga gaaaaatacc gc 22
<210> 3
   <211> 21
   <212> DNA
   <213> artificial sequence
<220>
   <223> primer
<400> 3 aaacgaacgc cgaaacacat a 21
<210> 4

   <211> 21
   <212> DNA
   <213> artificial sequence
<220>
   <223> primer
<400> 4
   gagcagattg ttttgagtag g 21
<210> 5
   <211> 20
   <212> DNA
   <213> artificial sequence
<220>
   <223> primer
<400> 5 ttcctaagca actcatcgtg 20
<210> 6
   <211> 24
   <212> DNA
   <213> artificial sequence
<220>
   <223> p314 primer
<400> 6 tgataacgat tttattgtcg cctc 24
<210> 7
   <211> 24
   <212> DNA
   <213> artificial sequence
<220>
   <223> p315 primer
<400> 7 tgatacagct agaaaattga acca 24
<210> 8
   <211> 24
   <212> DNA
   <213> artificial sequence
<220>
   <223> p316 primer
<400> 8 gaccccatac cacttctttt tgtt 24
<210> 9
   <211> 24
   <212> DNA
   <213> artificial sequence
<220>
   <223> p317 primer
<400> 9 tgatctcgtt ctagttctgg aagc 24
<210> 10
   <211> 21
   <212> DNA
   <213> artificial sequence
<220>
   <223> p325 primer
<400> 10 tcagcttctg ggcttggttc t 21
<210> 11
   <211> 21
   <212> DNA
   <213> artificial sequence
<220>
   <223> p326 primer
<400> 11 tcaacaccga acttggccaa t 21
<210> 12
   <211> 20
   <212> DNA
   <213> artificial sequence
<220>
   <223> p323 primer
<400> 12 taccggccaa atcgattctc 20
<210> 13
   <211> 22
   <212> DNA
   <213> artificial sequence
<220>
   <223> p324 primer
<400> 13 cactggtatt gttttggata cc 22
<210> 14
   <211> 21 <212> DNA
   <213> artificial sequence
<220>
   <223> p273 primer
<400> 14 aaagcttgtc caatcattgg c 21
<210> 15
   <211> 20
   <212> DNA
   <213> artificial sequence
<220>
   <223> p274 primer
<400> 15 ttttctctca caggtctgcg 20
<210> 16
   <211> 346
   <212> DNA
   <213> artificial sequence
<220>
   <223> 346 pb wild-type URE2 fragment
<400> 16
   aaagcttgtc caatcattgg cgcatgccct gacgtttgga agaacaacca tgcgttgatt    60
   tgtgattggt cagctaaatc atcggaccag agtaatggat taccagtctc tttgtaatat   120
   ttatttacca aatgtaataa aatcgcccct gattcccaaa tagacaagtt gtccatacca   180
   tgatcgatta aagctggaac tcttgcatta gggttcacag acacaaattc gggggcccta   240
   tgttcgccaa gattgaaatc taggaagatt gtgttataat gaaatccaag ttcacttagt   300
   actatagcaa ctttgaatcc attaggcgca gacctgtgag agaaaa                  346
<210> 17
   <211> 346
   <212> DNA
   <213> artificial sequence
<220>
   <223> 346 pb G182E URE2 mutant fragment
<400> 17
   aaagcttgtc caatcattgg cgcatgccct gacgtttgga agaacaacca tgcgttgatt    60
   tgtgattggt cagctaaatc atcggaccag agtaatggat taccagtctc tttgtaatat   120
   ttatttacca aatgtaataa aatcgcctct gattcccaaa tagacaagtt gtccatacca   180
   tgatcgatta aagctggaac tcttgcatta gggttcacag acacaaattc gggggcccta   240
   tgttcgccaa gattgaaatc taggaagatt gtgttataat gaaatccaag ttcacttagt   300
   actatagcaa ctttgaatcc attaggcgca gacctgtgag agaaaa                  346

## Description

The invention relates to the use of *URE2* mutant yeasts for increasing the release of aromatic volatile thiols by yeast during fermentation and its application for improving the aroma of fermented products, in particular alcoholic beverages produced by yeast fermentation (wine, cider, beer),as well as for developing yeast starter strains with optimized volathile thiol release capabilities.

Volatile thiols are extremely aromatic molecules that can affect the aroma of numerous foods such as fruits and grilled meat (Shankaranarayana *et al.,* Sulphur compounds in flavours. In Food Flavors; Morton, I.D., Macleod, A.J., Eds; Elsevier Science Publishers: Amsterdam, 1982; part A, pp169-281). Several compounds in this family, play a decisive role in contributing to the aroma of wines made from the Sauvignon Blanc grape variety (Tominaga et al., J. Agric. Food Chem., 1998c, 46: 5215-5219). Their aromatic contribution was also successively demonstrated in several other grape varieties such as Gewürztraminer, botrytized Sémillon, Colombard, Petit Manseng (Tominaga et al., Am. J. Enol. Vitic., 2000a, 51, 178-181), and Merlot (Aznar et al., J. Agric. Food Chem., 2000, 49, 2924-2929). Among these compounds, 4-mercapto-4methylpentan-2-one (4MMP) (Darriet et al., Flavour Fragr. J., 1995, 10, 385-392; Tominaga *et al.,* 1998c, precited) and 3-mercaptohexan-1-ol (3MH) (Tominaga et al., Flavour Fragr. J., 1998a, 13, 159-162) are principally formed during alcoholic fermentation from inodorous non-volatile specific S-cysteine conjugate precursors present in the must (Tominaga et al., J. Int. Sci. Vigne Vin., 1995, 29, 227-232; Tominaga *et al.,* 1998a). The transformation of such precursors in volatile thiols is achieved by yeast metabolism and involves a carbon-sulfur β-lyase activity (Tominaga *et al.,* 1998c ; Wakabayashi et al., J. Agric. Food Chem., 2004, 52, 110-116). The 3-mercaptohexyl acetate (3MHA or A3MH) resulting from the acetylation of 3MH by yeast also contributes to wine aroma.

4MMP, 3MHA, and 3MH are characterized by fruity descriptors (box tree, passion fruit, and grapefruit, respectively) and present very low perception thresholds (0.8, 4.2, and 60 ng L⁻¹, respectively; Tominaga *et al.,* 1998a). In addition, 3MH and 3MHA are chiral molecules with 2 enantiomers, *R* and S. In dry white wine made from healthy grapes, 3MH is almost racemic (R and S ratio around 50:50), whereas 3MHA is mainly in the S form (R and S ratio around 30:70). In wines made from botyrized grapes (*Botrytis cinerea*), the enantiomer distribution of 3MH is 30:70 in favor of the S form. It was recently demonstrated that 3MH(R) and 3MH(S) enantiomers produced different aromatic nuances (grape fruit and passion fruit, respectively) with similar perception thresholds in hydroalcoholic model solution (50 and 60 ng L, respectively). The perception thresholds of the R and S enantiomers of 3MHA are slightly different (9 and 2.5 ng/L): the less odoriferous R form is reminiscent of passion fruit, while the S form has a more herbaceous odor of boxwood (Tominaga et al., J. Agric. Food. Chem., 2006, 54, 7251-7255).

The powerful aromatic properties of these molecules prompted recent studies to understand how their production can be enhanced and modulated in winemaking. Viticultural practices such as nitrogen feeds (Choné et al., J. Int. Sci. Vigne Vin., 2006, 40, 1-6) and pre-fermentation operations such as skin contact (Peyrot des Gachons et al., Am. J. Enol., Vitic., 2002, 53, 144-156) modulate the amount of aromatic precursors in grape must. Fermentation conditions such as temperature (Howell et al., FEMS Microbiol. Letter, 2004, 240, 125-129; Masneuf-Pomarède et al., Int J. Food Microbiol., 2006, 108, 385-390) also influence the final concentration of these aromas in wine. However, the most important factor in thiol liberation is undoubtedly the yeast strain (Dubourdieu et al., Am. J. Enol. Vitic., 2006, 57, 81-88). A strong variability (about tenfold) was found among industrial wine starter strains of *S. cerevisiae* (Murat et al., 2001, Am. J. Enol., 52, 136-140; Howell *et al.,* 2004, precited; Masneuf-Pomarède et al., Int. J. Food Microbiol., 2006, 108, 385-390), *S. uvarum,* and related inter-specific hybrids (Masneuf et al., J. Int. Sci. Vigne Vin, 2002, 36, 205-212). This variability induced strain screening and breeding programs to obtain new strains having higher thiol liberation (Murat *et al.,* 2001; Masneuf *et al.,* 2002, precited). However, the genetic determinism and molecular mechanisms of thiol liberation by yeast remains unclear.

Recently, Howell *et al.* identified three genes *BNA3, CYS3,* and *IRC7* that encode putative β-lyase in the *Saccharomyces cerevisiae* genome. Using gene deletion experiments in a wine yeast strain background, they suggested that these enzymes are able to cleave 4MMP-precursor into the related volatile aroma during fermentation in synthetic grape juice (Howell et al., Applied Environ. Microbiology, 2005, 71, 5420-5426): Among these genes, *BNA3* encoding for an arylformamidase was found to contribute strongly to volatile thiol release. Although these enzymes have been identified, the mechanisms of precursor assimilation and cleavage by yeast during fermentation are far from being well-understood.

Ure2p is a transcriptional regulator of genes involved in nitrogen metabolism in yeast acting as negative regulator of Nitrogen Catabolite Repression (NCR)-sensitive genes (Cooper, T. G. and Sumrada, R.A., J. Bacteriol., 1983, 155, 623-627; Coschinago, P.W. and Magasanik, B., Mol. Cell. Biol., 1991, 11, 822-832; Ter Schure et al., FEMS Microbiol. Reviews, 2000, 24, 67-83; Magasanik, B., P.N.A.S., 2005, 102, 16537-16538; Cooper T., 1982, Transport in Saccharomyces cerevisiae. In The molecular biology of yeast Saccharomyces metabolism and gene expression. Strathern, J.N., Laboratory CSH, Eds, pp. 399-461)*.* Nitrogen Catabolite Repression (NCR) is the mechanism by which a yeast is able to select nitrogen sources enabling the best growth (preferred nitrogen source, good nitrogen source or rich nitrogen source : glutamate, glutamine, ammonia and asparagine) over non-preferred nitrogen sources (poorer nitrogen source : urea, gamma-amino-butyrate, amino acids such as proline and arginine), by repressing the transcription of some genes (NCR-sensitive genes) involved in the utilization (transport : permeases ; degradation : catabolic enzymes) of the poorer nitrogen sources (Cooper and Sumrada, precited). Under nitrogen-rich conditions (repressive conditions), Ure2p forms complexes with Gln3p and Gatlp, transcriptional activators of the GATA family, in the cytoplasm, to decrease the transcription of genes encoding the proteins needed to transport and degrade poor nitrogen sources (NCR-sensitive expression; Ter Schure *et al.,* precited; Scherens *et al.,* FEMS Yeast Res., 2006, 6, 777-791). On the other hand, when only non-preferred nitrogen sources are available, Gln3p and Gatlp are dephosphorylated , and move from the cytoplasm to the nucleus, where they positively regulate the expression of NCR-sensitive genes for nitrogen transport and catabolism. Nitrogen Catabolic Repression control is effective during wine fermentation (Rossignol et al., Yeast, 2003, 20, 1369-1385; Beltran et al., FEMS Yeast Res., 2004, 4, 625-632) and can be removed by deleting the *URE2* gene. Such deletion improves nitrogen uptake leading to an improvement in fermentation kinetics under enological conditions (Salmon, J.M. and Barre P., Applied and Environ. Microbiol., 1998, 64, 3831-3837; I.S. Pretorius, Yeast, 2000, 16, 675-729).

Ure2p displays a GST fold containing an N-terminal thioredoxin-fold domain and a C-terminal alpha helical domain. A transmissible conformational change of Ure2p results in a prion called [Ure3], an inactive, self-propagating and infectious amyloid. The N-terminal thioredoxin-fold domain (N-terminal ~ 90 amino acids) is sufficient to induce the [Ure3] phenotype and is also called the prion domain of Ure2p (Masison et al., P.N.A.S., 1997, 94, 12503-12508). In addition to its role in nitrogen regulation, Ure2p confers protection to cells against heavy metal ion and oxidant toxicity, and shows glutathione (GSH) peroxidase activity (Bai et al., J. Biol. Chem., 2004, 279, 50025-50030). The GST active site is located in a cleft between the N- and C-terminal domains.

The *URE2* gene that is conserved in yeast encodes the Ure2p-like subfamily of proteins composed of the *Saccharomyces cerevisiae* Ure2p of the amino acid sequence SEQ ID NO : 1 (354 amino acids, Accession number NP_014170.1) and related Glutahione S-Tranferases (GSTs) of other yeasts having a sequence that is homologous to SEQ ID NO : 1. For example, the *Saccharomyces cerevisiae URE2* gene is located from positions 219138 to 220202 on the complement strand of chromosome XIV (Accession number NCR_001146; gene ID 855492, locus tag YNL229C). The *URE2* gene sequence of other yeasts is available on the databases.

The *Saccharomyces cerevisiae IRC7* gene (GeneID: 850616 ; Systematic name : YFR055W) which is located from positions 264191 to 265213 on chromosome VI (Accession number : NCR_001138.4) encodes Irc7p : a cystathionine beta-lyase.

The inventors have constructed a *URE2* gene deletion mutant (Δ*ure2* mutant) in a wine yeast strain background and shown that, under enological conditions, the Δ*ure2* mutant releases 3 times as many volatile thiols as the wild type strain. Surprisingly, the 3MH (*R*)/(*S*) balance was increased significantly in the *Δure2* strain, putting the (*R*)/(*S*) ratio at 70:30, as compared to 50:50 in the wild type strain. These results indicate that, in addition to the enhancement of aroma release, Δ*ure2* deletion modifies the sensory perception of 3MH aroma of wine by intensifying the grapefruit overtones (3MH(R) descriptor).

In addition, contrary to what was suggested from previous reports (Howell *et al.,* 2005, precited), *IRC7, CYS3* and *BNA3* deletion experiments in a wine yeast background, indicated that Irc7p is the main enzyme involved in thiols precursor cleavage under enological conditions; Bna3p and Cys3p do not seem to play a pivotal role in the release of volatile thiols, under enological conditions. In addition, *IRC7* deletion experiments in a *Δure2* wine yeast background indicated that *IRC7* is the main gene activated by *URE2* deletion. Moreover, in the absence of Irc7p, the stoichiometric switch of (R)/(S)-3MH enantiomers induced by URE2 deletion was not observed. This result suggested that the Irc7p enzyme had a best substrate specificity for (*RR*)-P-3MH producing by consequence more (*R*)-3MH.

Thus, from a biotechnological point of view, these novel properties of *URE2* mutant yeasts and *IRC7* overexpressing yeasts open new avenues for consistently producing wine to definable specifications and style, as well as for developing wine yeast starter strains with optimized volatile thiol release capabilities.

The invention relates to the use of an *URE2* mutant yeast (*URE2* mutant) having a loss-of-function mutation in the *URE2* gene for increasing the release of aromatic volatile thiol by yeast during fermentation.

### Definition

- "mutation" refers to the substitution, deletion, addition of one or more nucleotides in a polynucleotide (cDNA, gene).
- *"**URE2* mutant having a loss-of function mutation in the *URE2* gene", refers to a yeast mutant which has a partial or total (null mutation) loss of function in the *URE2* gene and exhibits defects in nitrogen catabolite repression (NCR) and/or in glutathione peroxidase activity. The defect in NCR results in a mutant that is able to assimilate poorer nitrogen sources in the presence of good nitrogen sources. The defect in glutathione peroxidase activity increases the sensitivity to heavy metal ions and oxidants.
- by "homologous" is intended a sequence with enough identity to another one to lead to a protein having the same function, particularly having at least 70 % identity, preferably 80 % identity and more preferably 90 %.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.

- "wine yeast" refers to a yeast, usually a strain of *Saccharomyces sp.,* with high ethanol tolerance, resistance to sulfur dioxide and ability to rapidly and efficiently ferment grape juice containing 20 to 25 % sugar without producing organoleptic defects (acetate (volatile acidity)), hydrogen sulfide and phenol compounds).
- "aromatic volatile thiol" refers to an organic chemical compound that : (i) contains the functional group composed of a sulfur atom and a hydrogen atom (-SH), (ii) has high enough vapour pressures under normal conditions to significantly vaporize, and (iii) is odorous (flavour active).
- "fermentation" refers to any of a group of chemical reactions in which a living or non-living microorganism causes an organic substance to break down into simpler substances; especially, the anaerobic breakdown of sugar into alcohol (alcoholic fermentation).
- wine refers to an alcoholic beverage produced by the fermentation of the juice of fruits, usually grapes. Non-grape wines are called fruit wine or country wine. Other products made from starch based materials, such as barley wine, rice wine (sake), are more similar to beers. Beverages made from other fermentable material such as honey (mead), or that are distilled, such as brandy, are not wines.
- beer refers to an alcoholic fermented beverage most often made from malted barley, hops, yeast and water.

According to the present invention, the volatile thiol is released from non-volatile thiol precursor(s) present in a product (initial product), during fermentation by the *URE2* mutant. Fermentation of the initial product by the *URE2* mutant results in a fermented product (finished product), in particular an alcoholic beverage (wine, cider, beer), having improved aroma as a result of higher aroma active volatile thiol content.

According to the present invention, the improvement of the aroma includes the enhancement of the aroma resulting from the enhanced release of the different nuances of said aroma. It may also include the selective enhancement of a specific nuance of the aroma, resulting in a selective modification of the aroma.

The *URE2* mutant may be a natural mutant or a mutant obtained by genetic manipulation.

The mutant is derived from yeast strains able to ferment high amount of sugar including wine, brewery, bakery and distillation strains and/or any other strains well known for their ability to release aromatic compounds.

Natural mutants lacking Ure2p function (null alleles or null mutation) are well-known in the art ; for example *URE2* mutant alleles, also known as *usu* and *gdh*CR, have been described previously (Grenson, M., Eur. J. Biochem., 1969, 11, 249-260; Grenson et al., Mol. Gen. Genet., 1974, 128, 73-85 ; Courchesne et al., J. Bacteriol., 1988, 170, 708-713 ; Drillien et al., J. Bacteriol., 1972, 109, 203-208 (FL467-3D strain); Legrain et al., Eur. J. Biochem., 1982, 123, 611-616 (12597a strain); Brandriss et al., Can. J. Bot., 1995, 73 (Suppl.) : S153-S159 ; Coffman et al., J. Bacteriol., 1994, 176, 7476-7483 ; Xu et al., Mol. Cell. Biol., 1995, 15, 2321-2330; Coschigano, P.W. and B. Magasanik, Mol. Cell. Biol., 1991, 11, 822-832). These mutants were characterized as possessing: (i) nitrogen catabolic enzymes insensitive to nitrogen catabolite repression, mainly in the pathways involved in glutamate, glutamine, arginine, allantoin, urea, gamma-aminobutyrate and proline assimilation, (ii) increased amino acid permeases activity, (iii) resistance of ureidosuccinate transport to nitrogen repression. The *URE2* mutation was identified for some of the mutants. For example, the *ure2-1* mutation identified in the P5-5D strain of S. *cerevisiae* is a t to c transition at position 1119 of the nucleotide sequence causing the phenylalanine at position 313 of the amino acid sequence to be replaced by a serine (Coschigano, P.W. and Magasanik B., precited).

Other *URE2* mutants may be obtained by screening for yeasts having a phenotype as defined above.

*URE2* mutants having large deletions in the *URE2* coding sequence and lacking Ure2p function, have been constructed according to standard gene disruption techniques in yeast (Rothstein, R.J., Methods Enzymol., 1983, 101, 202-211 ; Wach et al., Yeast, 1994, 10, 1793-1808). For example, *URE2* disruption mutants were constructed by replacing one chromosomal copy of the *URE2* coding sequence, entirely or in part (SacII-PvuII or *Hind*III fragments), by a complementing selection marker for yeast *(LEU2, URA3).*

*URE2* mutants were constructed by random mutagenesis (UV mutagenesis) and screening for yeasts derepressed for proline utilization under nitrogen repression, able to grow in the presence of proline as the sole nitrogen source and methylamine (ammonium analog) at repressive concentration (Salmon, J.M. and Barre P., Applied and Environmental Microbiology, 1998, 64, 3831-3837).

Other mutants may be constructed by standard mutagenesis (random and site-directed) techniques which are well-known in the art, followed by screening for *URE2* mutants having a phenotype as defined above.

The *URE2* mutant may be homozygous or heterozygous for the *URE2* mutation.

In addition, the *URE2* mutation may be introduced in an appropriate background by spore to spore mating and segregation analysis using conventional yeast genetic methods using homothallic or heterothallic strains (US Patent 6,140,108; Marullo et al., FEMS Yeast Res., 2006, 6, 268-279; Bakalinsky et al., Applied and Environ. Microbiology, 1990, 56, 849-857).

The release of volatile thiol is measured by an appropiate method, well-known in the art, such as for example : specific extraction of volatile thiols (Tominaga et al., J. Agric. Food. Chem., 1998, 46, 1044-1048; Tominaga et al., J. Agric. Food. Chem., 2000, 48, 1799-1802), followed by gas chromatography coupled with mass spectrometry (GC/MS; Tominaga et al., J. Agric. Food. Chem., 1998, 46, 1044-1048), flame photometric detection (GC/FPD; Tominaga, T. and Dubourdieu, D., Flavour Fragrance J., 1997, 12, 373-376), olfactometry (GC/olfactometry; Darriet et al., J. Int. Sci. Vigne Vin., 1991, 25, 167-174) or atomic emission detection (GC/AED; Howell et al., FEMS Microbiology Letters, 2004, 240, 125-129).

The increase of volatile thiol by the *URE2* mutant may be evaluated by comparison with a nearly isogenic yeast strain not mutated in the *URE2* gene, grown in the same conditions.

According to a preferred embodiment of said use, the *URE2* mutant is a null mutant lacking Ure2p function.

According to another preferred embodiment of said use, the URE2 mutant yeast is homozygous for the *URE2* mutation.

According to another preferred embodiment of said use, the *URE2* mutant is a mutant of *Saccharomyces sp.* or of a derived interspecific hybrid. In particular, the mutant may be derived from *S*. *cerevisiae, S. bayanus (var. uvarum), S*. *paradoxus* or from interspecific hybrids of *Saccharomyces,* such as *S*. *pastorianus.*

According to another preferred embodiment of said use, the *URE2* mutant is a mutant of a wine yeast, preferably of a commercial wine yeast strain chosen from:VL3c, X5 and RX60.

According to another preferred embodiment of said use, the volatile thiol derives from a S-cysteine conjugate precursor, as indicated in Table I.

**Table I: Volatile thiols and their precursor**

| **Volatile thiol** | **Precursor** |
|---|---|
| 4-mercapto-4-methylpentan-2-one (4MMP) | S-4-(4-methylpentan-2-one)-L-cysteine |
| 4-mercapto-4-methylpentan-2-ol (4MMPOH) | S-4-(4-methylpentan-2-ol)-L-cysteine |
| 3-mercaptohexan-l-ol (3MH) | S-3-(hexan-l-ol)-L-cysteine |
| 3-mercaptohexyl acetate (A3MH or 3MHA) | 3MH |
| 3-mercaptopentan-1-ol (3MPOH) | S-3-(pentan-1-ol)-L-cysteine |
| 3-mercaptoheptan-1-ol (3MHepOH) | S-3-(heptan-1-ol)-L-cysteine |
| 3-mercapto-2-methylbutan-1-ol (3M2MBOH) | S-3-(2-methylbutan-2-ol)-L-cysteine |
| 3-mercapto-2-methylpentan-1-ol (3M2MPOH) | S-3-(2-methylpentan-2-ol)-L-cysteine |

| | |
|---|---|
| *A3MH is the product of 3MH esterification | |

The volatile thiol is preferably selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

Furthermore, the 3MH which is released by the yeast mutant may advantageously consist of more *(R)* enantiomer than *(S)* enantiomer; the 3MH consists advantageously of at least 70 % of *(R)* enantiomer (*(R)* to *(S)* enantiomer ratio superior or equal to 70:30).

According to another preferred embodiment of said use, the release of at least two different aromatic volatile thiols is increased during fermentation.

According to another preferred embodiment of said use, the release of aromatic volatile thiol is increased at least by a factor of 3.

According to another preferred embodiment of said use, said fermentation is alcoholic fermentation, preferably wine fermentation.

The invention relates also to a method for enhancing the aroma of a fermented product produced by fermentation of an initial product containing at least one non-volatile thiol precursor, comprising the incubation of the initial product with a *URE2* mutant yeast as defined above, under conditions allowing fermentation by the *URE2* mutant to occur, and thereby increase the release of the volatile thiol(s)-from the non-volatile thiol precursor(s)-, by the mutant yeast, during fermentation.

According to a preferred embodiment of said method the fermented product is an alcoholic beverage, preferably wine, more preferably wine made from a simple or non-floral grape variety, such as for example: Sauvignon Blanc, Gewürztraminer, Riesling, Semillon, Colombard, Scheurebe, Petit Manseng, Cabernet Sauvignon and Merlot.

According to another preferred embodiment of said method the non-volatile thiol precursor is a S-cysteine conjugate precursor.

The volatile thiol is preferably selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

According to another preferred embodiment of said method, the release of aromatic volatile thiol(s) is increased at least by a factor of 3.

The invention relates also to a method for enhancing the grapefruit overtone of a fermented product produced by fermentation of an initial product containing S-3-(hexan-1-ol)-L-cysteine (3MH precursor), comprising the incubation of the initial product with a *URE2* mutant yeast as defined above, under conditions allowing fermentation by the *URE2* mutant to occur, and thereby increase the release of the 3MH (R) enantiomer, by the mutant yeast, during fermentation.

According to a preferred embodiment of said method, the fermented product is an alcoholic beverage, preferably wine, more preferably wine made from a simple or non-floral grape variety, such as for example: Sauvignon Blanc, Gewürztraminer, Riesling, Semillon, Colombard, Scheurebe, Petit Manseng, Cabernet Sauvignon and Merlot.

According to a preferred embodiment of said method, the 3MH *(R)* to *(S)* enantiomer ratio is superior or equal to 70:30 (3MH consisting of at least 70 % of *(R)* enantiomer)

The invention relates also to a method for identifying yeasts able to improve the aroma of a fermented product, comprising at least the steps of:
(a) obtaining *URE2* mutant yeasts,
(b) inoculating the *URE2* mutant(s) of step (a) in a fermentation medium containing non-volatile thiol precursor(s), under conditions allowing fermentation by the *URE2* mutant to occur, and thereby release of the volatile thiol(s)-from the non-volatile thiol precursor(s)-, by the mutant yeast, during fermentation,
(c) measuring the volatile thiol(s) released in the fermented medium, by any appropriate means, as defined above,
(d) identifying the *URE2* mutant(s) able to release high level aromatic volatile thiol, as compared to yeast having a wild-type *URE2* gene in a nearly isogenic genetic background.

The *URE2* mutants are derived from a yeast strain able to ferment high amount of sugar including wine, brewery, bakery and distillation strains and/or any other strain well known for its ability to release aromatic compounds.

The *URE2* mutants may be obtained by screening *URE2* mutant(s) from a yeast collection or by genetic manipulation, according to standard methods, as defined above.

According to a preferred embodiment of said method, the *URE2* mutant is derived from a strain of *Saccharomyces sp.* or of a derived interspecific hybrid, as defined above.

According to another preferred embodiment of said method, the *URE2* mutant is derived from a wine yeast strain, preferably chosen from VL3c, X5 and RX60.

According to another preferred embodiment of said method, the fermentation of step (b) is alcoholic fermentation.

According to another preferred embodiment of said method, step (c) comprises the measurement of volatile thiol(s) selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

Preferably, the *URE2* mutant identified in step (d) releases at least twice, preferably at least three times more volatile thiol than a yeast having a wild-type *URE2* gene in the same genetic background.

Since Irc7p is the main enzyme involved in volatile thiols release under oenological conditions, *IRC7* overexpressing yeasts have a potential to improve the aroma of a fermented product.

The invention relates also to a method for screening yeasts having a potential to improve the aroma of a fermented product, comprising at least the steps of:
(a) providing a collection of yeasts,
(b) assaying *IRC7* expression level in said yeasts during alcoholic fermentation,
(c) identifying the yeasts having high *IRC7* expression level compared to a *URE2* mutant yeast of a nearly isogenic genetic background.

The yeasts are yeast strains able to ferment high amount of sugar including wine, brewery, bakery and distillation strains and/or any other strain well known for its ability to release aromatic compounds.

According to a preferred embodiment of said method, the yeasts are of *Saccharomyces sp.* or of a derived interspecific hybrid, as defined above.

According to another preferred embodiment of said method, the yeasts are wine yeast strains.

The *IRC7* expression level is assayed by methods which are well-known in the art. For example *IRC7* transcription may be measured by quantitative real time PCR using standard protocols which are known in the art.

According to another preferred embodiment of said method, it comprises the further steps of :
(d) inoculating the *IRC7* overexpressing yeasts of step (c) in a fermentation medium containing non-volatile thiol precursor(s), under conditions allowing fermentation by the *IRC7* overexpressing yeasts to occur, and thereby release of the volatile thiol(s)-from the non-volatile thiol precursor(s)-, by the *IRC7* overexpressing yeasts, during fermentation,
(e) measuring the volatile thiol(s) released in the fermented medium, by any appropriate means, as defined above,
(f) identifying the *IRC7* overexpressing yeasts able to release high level aromatic volatile thiol compared to a *URE2* mutant yeast of a nearly isogenic genetic background.

According to another preferred embodiment of said method, step (e) comprises the measurement of volatile thiol(s) selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

Preferably, the *IRC7* overexpressing yeasts identified in step (f) release at least twice, preferably at least three times more volatile thiol than a yeast having a wild-type *URE2* gene in a nearly isogenic genetic background.

Since Irc7p is the main enzyme involved in volatile thiols release under oenological conditions, *IRC7* overexpressing yeasts may replace the *URE2* mutant yeasts in the use and the methods as defined above.

The invention relates also to the use of an *IRC7* overexpressing yeast for increasing the release of aromatic volatile thiol by yeast during fermentation.

According to another preferred embodiment of said use, the volatile thiol derives from a S-cysteine conjugate precursor, as indicated in Table I.

The volatile thiol is preferably selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

Furthermore, the 3MH which is released by the yeast may advantageously consist of more *(R)* enantiomer than *(S)* enantiomer; the 3MH consists advantageously of at least 70 % of *(R)* enantiomer (*(R)* to *(S)* enantiomer ratio superior or equal to 70:30).

According to another preferred embodiment of said use, the release of at least two different aromatic volatile thiols is increased during fermentation.

According to another preferred embodiment of said use, the release of aromatic volatile thiol is increased at least by a factor of 3.

According to another preferred embodiment of said use, said fermentation is alcoholic fermentation, preferably wine fermentation.

The invention relates also to a method for enhancing the aroma of a fermented product produced by fermentation of an initial product containing at least one non-volatile thiol precursor, comprising the inoculation of the initial product with an *IRC7* overexpressing yeast, under conditions allowing fermentation by the *IRC7* overexpressing yeast to occur, and thereby increase the release of the volatile thiol(s)-from the non-volatile thiol precursor(s)-, by the *IRC7* overexpressing yeast, during fermentation.

According to a preferred embodiment of said method the fermented product is an alcoholic beverage, preferably wine, more preferably wine made from a simple or non-floral grape variety, such as for example: Sauvignon Blanc, Gewürztraminer, Riesling, Semillon, Colombard, Scheurebe, Petit Manseng, Cabernet Sauvignon and Merlot.

According to another preferred embodiment of said method the non-volatile thiol precursor is a S-cysteine conjugate precursor.

The volatile thiol is preferably selected from the group consisting of: 4MMP, 3MH, 4MMPOH, 3MHA, 3MPOH, 3MHepOH, 3M2MBOH, and/or 3M2MPOH, more preferably from 4MMP, 3MH and/or 3MHA.

According to another preferred embodiment of said method, the release of aromatic volatile thiol(s) is increased at least by a factor of 3.

The invention relates also to a method for enhancing the grapefruit overtone of a fermented product produced by fermentation of an initial product containing S-3-(hexan-1-ol)-L-cysteine (3MH precursor), comprising the inoculation of the initial product with an *IRC7* overexpressing yeast as defined above, under conditions allowing fermentation by the *IRC7* overexpressing yeast to occur, and thereby increase the release of the 3MH (R) enantiomer, by the *IRC7* overexpressing yeast, during fermentation.

According to a preferred embodiment of said method, the fermented product is an alcoholic beverage, preferably wine, more preferably wine made from a simple or non-floral grape variety, such as for example: Sauvignon Blanc, Gewürztraminer, Riesling, Semillon, Colombard, Scheurebe, Petit Manseng, Cabernet Sauvignon and Merlot.

According to a preferred embodiment of said method, the 3MH *(R)* to *(S)* enantiomer ratio is superior or equal to 70:30 (3MH consisting of at least 70 % of *(R)* enantiomer)

The *IRC7* overexpressing yeast may be obtained by screening of a yeast collection as defined above or by genetic manipulation, according to standard methods.

The practice of the present invention will employ, unless otherwise indicated, conventional techniques of microbiology, recombinant DNA, molecular biology, cell biology and cell culture which are within the skill of the art. Such techniques are explained fully in the literature. See, for example, Methods of classical genetics. Biotechnology Handbooks Saccharomyces. Vol 4. (Wickner, R, Truite M. & Oliver S., Eds, 1991, pp 101-147); Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press).

In addition to the preceding features, the invention further comprises other features which will emerge from the description which follows, which refers to examples illustrating the use of *URE2* mutants according to the invention, as well as to the appended drawing in which:

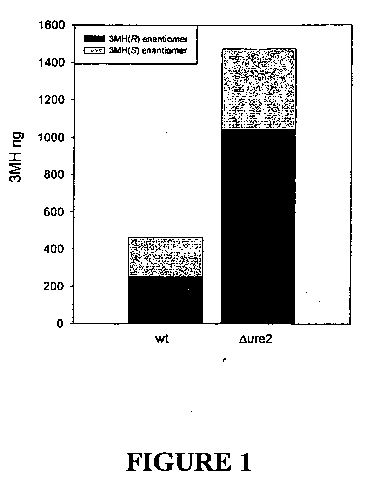

- figure 1 illustrates the stereo-selectivity of Δ*ure2* mutants for 3MH enantiomers. In the Δ*ure2* deletion strain, 3MH production was strongly increased compared to control; moreover, the 3MH*(R)*/*(S)* ratio was largely unbalanced, with 70% of 3MH*(R)*. Values are the average of triplicate experiments.

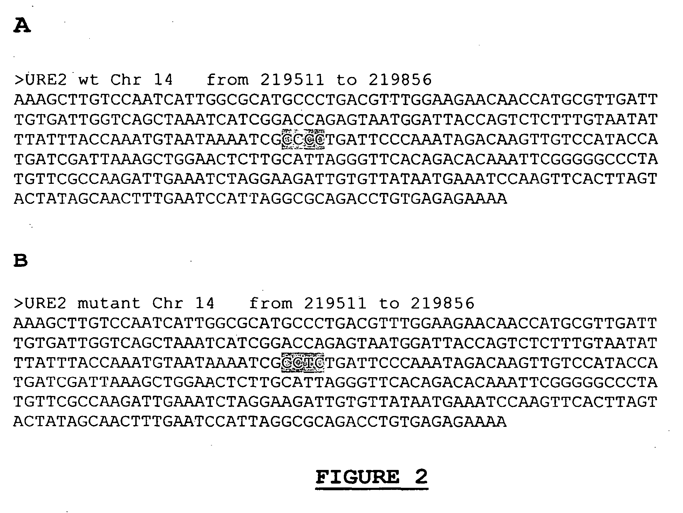

- figure 2 represents the sequence of a 346 bp fragment of the wild-type *URE2* gene (S288c strain; SEQ ID NO: 16) by comparison with that of an *URE2* defective allele carrying the G182E mutation (SEQ ID NO : 17). The 346 bp fragment corresponds to positions 219511 to 219856 of NCBI sequence accession number NC_001146. The mutation introduces a *Mnl1* site (cctc; highlighted in grey) in the mutant *URE2* allele that can be detected by PCR/RFLP *(Mnl1* cleavage occurs at position 156 of the fragment).

### Example 1: URE2 deletion improves thiol release under enological conditions

### 1) Material and methods

### a) Culture conditions and fermentation

Yeasts were grown at 30°C on complete YPD medium (1 % yeast extract, 1 % peptone, and 2 % dextrose) solidified with 2 % agar if necessary. G418 (100 µg ml⁻¹) was added to YPD to select transformed strains. Sporulation was induced on acetate medium (1 % potassium acetate, 2 % agar) for three days at 24°C. Fermentation experiments were carried out at 24°C in 350 ml-bottles containing a synthetic model medium previously described as KP medium (Marullo et al., FEMS Yeast Research, 2006, 6, 268-279). This medium was buffered to pH 3.3 and contained 80 g L⁻¹ glucose, 80 g L⁻¹ fructose, and 190 mg L⁻¹ available nitrogen. Aroma precursors (P-4MMP and P-3MH) were added to the medium before yeast inoculation at different concentrations, as indicated in the text. Yeast strains were inoculated at 1.10⁶ cells ml⁻¹ from an overnight pre-culture. Volatile thiol release was measured after complete fermentation (measured by weight loss). All experiments were carried out in triplicate and independently repeated two or three times.

### b) Yeast strains

*Saccharomyces cerevisiae* strain VL3-1D was derived from a commercial starter (VL3c, Laffort Oenologie). VL3-1D, a homothallic diploid spore clone (*HO*/*HO*) obtained by tetrad micro-dissection, is assumed to be totally homozygous (Marullo et al., FEMS Yeast Research, 2004, 4, 711-719). The Δ-U (*Δure2*/*Δure2*), Δ-B (Δ*bna3*/*Δbna3*), *Δcys3*/*Δcys3* and *Δirc7*/*Δirc7* strains were constructed from the VL3-1D strain using PCR deletion strategy (Baudin et al., Nucleic Acids Research, 1993, 21, 3329-330; Wach et al., Yeast, 1994, 10, 1793-1808). Deletion cassettes were obtained by amplifying the genomic DNA of appropriate deleted strains (BY4742 background) from the Euroscarf collection (Frankfurt, Germany). The *ure2::KanMx4* and *bna3::KanMx4* cassettes were obtained using p59: 5'-caaatgccgagaaaaataccgc-3' (SEQ ID NO: 2) and p60: 5'-aaacgaacgccgaaacacata-3'(SEQ ID NO: 3), pl: 5'-gagcagattgttttgagtagg-3'(SEQ ID NO: 4), p2: 5'-ttcctaagcaactcatcgtg-3' (SEQ ID NO: 5) primer pairs, respectively. The *irc7::KanMx4* and *cys3::KanMx4* cassettes were obtained using p314: 5'-tgataacgattttattgtcgcctc-3'(SEQ ID NO : 6) and p315: 5'-tgatacagctagaaaattgaacca-3' (SEQ ID NO : 7); p316: 5'-gaccccataccacttctttttgtt -3' (SEQ ID NO : 7) and p317: 5'-tgatctcgttctagttctggaagc-3' (SEQ ID NO : 8) primer pairs, respectively.

Cells were chemically transformed (Puig et al., J. Agric. Food Chem., 1998, 46, 1689-1693) and positively selected on YPD-G418. G418-growing clones were sporulated and micro-dissected in order to obtain fully homozygous spore clones carrying two deleted gene copies. Correct deletions were verified by PCR.

### c) Precursor synthesis

### P-4MMP synthesis

*S*-4-(4-methylpentan-2-one)-L-cysteine (P-4MMP) was synthesized by the addition of mesityl oxide (22 mmol) in L-cysteine (20 mmol) in distilled water for 16 h. After extraction with diethyl ether (2 x 30 ml), the aqueous layer was cooled to 0°C and di-tert-butyl dicarbonate (4.6 g, 21 mmol) in 20 ml of THF was added dropwise and stirred overnight. After elimination of THF by rotary evaporation, the aqueous layer was adjusted to pH 2-3 with 10 % citric acid in water and extracted with EtOAc (3 x 100 ml). Combined organic layers were washed with water (50 ml) and brine (50 ml), dried over magnesium sulfate, filtered, and concentrated under reduced pressure. The purification of the crude product by silica gel chromatography (pentane/ diethyl ether - 35/65, Rf = 0.5) gave a colorless oil (4.5 g, 70 %). 1 ml of aquous HCl 10M (10 mmol) was added to a *N*-(*tert*-Butoxycarbonyl)-*S*-4-(4-methylpentan-2-one)-L-cysteine (0.63g, 2 mmol) at room temperature. After reaction was complete, excess HCl was removed by heating at 60°C under reduced pressure (0.1 mm Hg).

### P-3MH synthesis

S-3-(hexan-1-ol)-L-cysteine (P-3MH) was synthesized according to (Wakabayashi et al., J. Agric. Food Chem., 2004, 52, 110-116) and modified as follows: *Trans*-2-hexen-1-al (5 mmol) was added to a stirred solution of N-(*tert-*Butoxycarbonyl)-L-cysteine (4.5 mmol) and cesium carbonate (2.25 mmol) in acetonitrile overnight. After elimination of acetonitrile by rotary evaporation, sodium borohydride (2.5 mmol) in 10 ml of methanol was added dropwise and stirred overnight. The deprotection of 0.5 ml of N-(*tert*-Butoxycarbonyl)-*S*-3-(hexan-1-ol)-L-cysteine was carried out by the addition of HCl 10M (5 mmol). The crude product was concentrated and purified on an ion exchange column (DOWEX 50 WX8).

### d) Volatile thiol assays

Volatile thiols were extracted at the end of fermentation from 250 ml of synthetic medium using the previously described method (Tominaga et al., J. Agric. Food Chem., 1998b, 46, 1044-1048) and modified according to (Tominaga et al., J. Agric. Food Chem., 2000b, 48, 1799-1802). Amounts of 4MMP, 3MH and 3MHA were quantified by GC-MS (Gas chromatography and solid-phase microextraction) according to methods described by Tominaga *et al.,* 1998b.

### 2) Results

The VL3-1D strain is a (*HO*/*HO*) meiotic spore clone of the commercial strain VL3c previously described for its strong capacity to reveal volatile thiols (Murat et al., Am. J. Enol. Vitic., 2001, 52, 136-140; Howell et al., FEMS Microbiol Lett., 2004, 240, 125-129; Howell et al., Appl. Environ. Microbiol., 2005, 71, 5420-5426; Masneuf-Pomarède et al., Int. J. Food Microbiol., 2006, 108385-390). The Δ-U strain carrying two deleted copies of *URE2* (*Δure2*/*Δure2*) was obtained from VL3-1D by *ure2::KanMx4* cassette mediated deletion.

The kinetic properties of this mutant were compared to the wt strain using bioreactors as previously described (Marullo et al., FEMS Yeast Research, 2006, 6, 268-279). Under enological conditions, both strains showed identical kinetic parameters.

Volatile thiol release of Δ*ure2* and *wt* strains was then assayed in 350 ml-batch cultures using a synthetic grape medium with the addition of synthetic precursors. Precursor concentrations were adjusted to 20 nM for P- 4MMP and 2000 nM for P-3MH in accordance with standard Sauvignon Blanc must composition (Peyrot des Gachons et al., J. Agric. Food Chem., 2000, 48, 3387-3391). During fermentation, yeast metabolism produced 4-mercapto-4-methylpentan-2-one (4MMP) from its *S*-cysteine conjugate (*S*-4-(4-methylpentan-2-one)-L-cysteine) henceforth referred to as P-4MMP. Similarly, 3-mercaptohexanol (3MH) was produced from *S-*3((hexan-1-ol)-L-cysteine (henceforth referred to as P- 3MH). A part of 3MH can also be acetylated during fermentation, producing 3-mercaptohexyl acetate (3MHA). At the end of the fermentation, 4MMP, 3MH, and 3MHA produced by yeast were organically extracted and measured by GC-MS (Tominaga et al., Flavour Fragr. J., 1998a, 13, 159-162). The absence of volatile thiols in unfermented control was previously demonstrated (Murat et al., Am. J. Enol. Vitic., 2001, 52, 136-140). As shown in Table II, *Δure2* mutant released 3 times as many volatile thiols (4MMP, 3MH, and 3MHA) as the wild type strain.

**Table II: URE2-deletion enhances volatile thiol release under enological conditions**

| Strains | Aromas released (ng L⁻¹)^{a} | | |
|---|---|---|---|
| | 4MMP | 3MH | 3MHA |
| VL3-1D (wt) | 30.2 +/- 1.9 | 467.5 +/- 55.4 | 85.9 +/- 21.9 |
| Δ-U (Δure2) | 87.6* +/- 8.5 | 1473.2* +/- 147.2 | 276.2* +/-14.5 |

| | | | |
|---|---|---|---|
| ^{a} Aromas were measured at the end of alcoholic fermentation of synthetic medium containing aroma precursors: P-4MMP=20 nM, P-3MH 2000 nM. Values are the means of two independent experiments carried out in triplicate. * Significantly different from control, ANOVA p value <0.001 | | | |

### Example 2: URE2 deletion modifies 3MH enantiomer balance

### 1) Material and methods

### Separation of two 3MH enantiomers

Extracted volatile thiols were injected into a Lipodex C (50 m × 0.25 mm) chiral column (INTERCHIM) to separate the two 3MH enantiomers. Chromatography conditions were identical to those described by Tominaga et al. (J. Agric. Food Chem., 2006, 54, 7251-7255).

### 2) Results

3MH is a chiral molecule with 2 enantiomers, 3MH(*R*) and 3MH(*S*). Using a specific GC-MS methodology (Tominaga et al., J. Agric. Food. Chem., 2006, 54, 7251-7255), R and S-3MH were measured in synthetic medium at the end of alcoholic fermentation. The relative amount of 3MH enantiomers of VL3-1D (wt) and Δ-U (*Δure2*) strains are shown in Figure 1. As generally observed in dry white wine, the medium fermented by VL3-1D (*wt*) presented a racemic mix between 3MH (*R*) and (*S*) enantiomers (50:50). Surprisingly, the *(R)* and (*S*)-forms in the Δ*ure2 strain* increased significantly (4.1 times and 2.0 times respectively), putting the (*R*)/(*S*) ratio at 70:30. This 3MH (*R*)/(*S*) balance modification suggests that stereo-selective mechanisms occurred in *Δure2* mutants. These findings indicated that, in addition to the enhancement of aroma release, *Δure2* deletion might modify the sensory perception of 3MH aroma of wine by intensifying the grapefruit overtones (3MH(*R*) descriptor).

These findings demonstrate that the *URE2* gene strongly inhibits the volatile thiol liberation mechanisms during alcoholic fermentation, probably involving the Nitrogen Catabolic Repression. Due to the upstream regulation of *URE2,* several regulated targets may affect thiol release. The full activation of Gln3p and Gatlp transcription factors may enhance the expression of two main gene categories. First, up-regulated genes encoding nitrogen catabolic enzymes (Scherens et al., FEMS, Yeast Research, 2006, 6, 777-791) may contribute to enhancing the pool of precursor cleaving enzymes. Second, expressed poor-nitrogen transporters may increase precursor uptake due to its amino acid-like structure.

### Example 3: Impact of several yeast β-lyases on volatile thiols release in function of precursor concentration

### 1) Material and methods

See example 1

### 2) Results

Three genes (*BNA3, CYS3* and *IRC7*) were described to strongly contribute to volatile thiols release (Howell et al., Appl. Environ. Microbiol., 2005, 71, 5420-5426). Since *URE2* deletion seems to mainly increase enzymatic cleavage activity, the role of these three genes and their regulation by NCR in enological conditions was investigated in depth. The two copies of *BNA3, CYS3,* and *IRC7* genes were deleted in VL3-1D background. The deletion effect of these genes was firstly assayed by measuring volatile thiols release at the end of the alcoholic fermentation process of a synthetic grape juice containing natural amounts of aroma precursors (*i.e.* P-4MMP = 20 nM and P-3MH = 3000 nM). At these concentrations, *IRC7* deletion drastically reduced 3MH (-41%) and 4MMP (-96%) release (Table III) while *BNA3* and *CYS3* deletion did not affect thiols release. This result partially contradicts those presented by (Howell et al., Appl. Environ. Microbiol., 2005, 71, 5420-5426)) that showed a role for *CYS3, BNA3* and *IRC7* genes in 4MMP release. However, their conclusions were based on experimental conditions in which synthetic medium contained 0.1 g L⁻¹ (450 µM) of P-4MMP, while in natural grape juice only a few µg L⁻¹ are found (0.5 to 20 nM) Peyrot des Gachons et al., J. Agric. Food Chem., 2000, 48, 3387-3391). It was hypothesized that these high concentrations could bias the stoichiometric conditions of β-lyase reaction. To verify this hypothesis, 4MMP release was measured in synthetic model medium containing 200,000 nM of P-4MSP, corresponding to 10,000 times the P-4MMP concentration in grape juice. In accordance with Howell's findings, at this concentration, the 4MMP release dropped nearly by 81% and 96% for Δ*bna3* and Δ*irc7* strains, respectively (Table III). However, deletion of *CYS3* gene did not affect this release even when using high concentration of P-4MSP.

**Table III : The cystathionine β-lyase Irc7p is the main enzyme involved in thiols precursor cleavage**

| Strains | precursor concentrations | | | | | |
|---|---|---|---|---|---|---|
| | P-4MMP 20 nM (1X) | | P-4MMP 200 µM (10,000X) | | P-3MH 2,000 nM (1X) | |
| | 4MMP released | | 4MMP released | | 3MH released | |
| | mean | CV (%) | mean | CV (%) | mean | CV (%) |
| *WT* | 100.0 | 5.6 | 100.0 | 5.0 | 100.0 | 2.7 |
| *Δcys3* | 92.5 | 6.0 | 97.1 | 1.9 | 104.5 | 6.5 |
| *Δirc7* | 3.6** | 21.1 | 1.7** | 0.0 | 58.5 ** | 9.7 |
| *Δbna3* | 115.5 | 5.7 | 32.3 ** | 15.1 | 91.7 | 7.3 |

| | | | | | | |
|---|---|---|---|---|---|---|
| **Statistically different from WT strain, ANOVA P= 0.01. | | | | | | |

### Example 4 : The enhancing effect of URE2 deletion is mediated by Irc7p expression.

### 1) Material an methods

### a) RNA extraction and cDNA synthesis

Biomass samples for expression assay were collected for 10, 20, 40 and 60 % of total CO₂ released. Ten-milliliter samples were centrifuged (5 min, 10 000 g) to pellet the cells and complete extraction was further achieved according to the manufacturer's instructions. Lysis treatment was performed using a Fastprep FP120 apparatus with the following parameters: 45 s at 65 m s⁻¹ applied twice. The two cycles were separated by 5 min on ice. DNA contamination was treated using DNA-free Kit (AMBION INC), according to the manufacturer's instructions. The absence of contaminating DNA was checked by PCR directly on the RNAs. RNAs were retro-transcribed into cDNAs using the iScriptTM cDNA Synthesis Kit (BIO-RAD), according to the manufacturer's instructions. For both strains (Δ*ure2* and *WT*) extractions were realized from three independent batches.

### b) IRC7 expression analysis by real time PCR

Quantitative real time PCR with SYBR-Green I was performed using the iCycler iQ real time PCR Detection System (BIO-RAD). Primers p325 (5'-tcagcttctgggcttggttct-3', SEQ ID NO: 10) and p326 (5'-tcaacaccgaacttggccaat-3', SEQ ID NO: 11.), and p323 (5'-taccggccaaatcgattctc-3', SEQ ID NO: 12) and p324 5'-cactggtattgttttggatacc-3', SEQ ID NO: 13) were used to amplify 137 bp of *IRC7* (gene of interest), and 124 bp of *ACT1* (housekeeping gene), respectively. The use of *ACT1* as housekeeping gene has been routinely described in the literature (Giulietti et al., Methods, 2001; 25, 386-401 ; Bleve et al., Appl. Environ. Microbiol., 2003, 69, 4116-4122) as quantification marker for many yeast species through quantitative real time PCR. Real time qPCRs were carried out by using iQ SYBR Green Super Mix (BIO-RAD). Primers were added at a concentration of 0.3 mM each. PCR program was as follows: 3 min at 95°C for initial denaturation, then 40 cycles of 30 s at 95°C during, 30 s at 58°C and 30 s at 72°C. A final melt curve was carried out by 51 cycles of 10 s starting a 65°C with increasing steps of 0.5°C at each cycle. The efficiency was 97.9 % and 102.8 % for *IRC7* and *ACT1,* respectively. A standard curve was determined for each gene where x is the Threshold Cycle and y is the LOG value of starting quantity (ng): (*IRC7 y=* -3.374 x + 19.132, R²=0.997 and *ACT1 y=* -3.257 x + 20.308, R²=0.993). Standard curves were obtained from 8 points and the linearity was observed from 0.15 pg to 63 ng of DNA. A threshold value for the fluorescence of all samples was set manually, to maintain the same value in each experiment. For strain comparison, relative amounts of *IRC7* and *ACT1* were calculated by report on standard curve. Results were given as the relative fold expression of *(IRC7*/*ACT1)* taking as reference (=1) the *wild type* value at 10% of CO₂ produced.

### 2) Results

The activation of *IRC7 (YFR055c)* transcription in a Δ*ure2* background was recently demonstrated in laboratory conditions (Scherens et al., Yeast Research, 2006, 6, 777-791). This transcription activation during the alcoholic fermentation was investigated for the wine strain of this study, using real time PCR.

**Table IV : IRC7 expression is enhanced during the alcoholic fermentation in a Δure2 strain.**

| *Strains* | Relative fold expression *(IRC7*/*ACT1)* at different % of CO₂ produced | | | | |
|---|---|---|---|---|---|
| | 10.0 | 20.0 | 40.0 | 60.0 | 80.0 |
| *WT* | 1.00 +/- 0.94 | 1.41 +/- 0.80 | 1.64 +/-0.44 | 1.18 +/- 0.34 | 0.93 +/- 0.31 |
| Δ*ure2* | 0.67 +/- 0.58 | 1.18 +/- 0.06 | 3.36* +/-0.52 | 2.36* +/-0.60 | 6.40* +/- 2.48 |

| | | | | | |
|---|---|---|---|---|---|
| *Statistically different from *WT* strain, ANOVA P= 0.05. | | | | | |

During the alcoholic fermentation, *IRC7* transcription level increased strongly in the Δ*ure2* strain as compared to the control *(WT)* (Table IV). The *IRC7* transcript levels were found significantly different after 40 % of CO₂ release (one-way ANOVA, P=0.05). Although smaller, this 2-fold increase in *IRC7* expression in the *Δure2* strain should explain the observed 3-fold overproduction of thiols. Nevertheless, other uninvestigated enzymes, among which Irc7p, might be activated by *URE2* deletion and further then contribute to the enzymatic cleavage. Therefore, the Irc7p contribution was investigated in a *Δure2* background by comparing thiols release in *WT, Δure2, Δicr7* and the double mutant Δ*ure2* Δ*irc7* (Table V).

**Table V : IRC7 is the main gene activated by URE2 deletion**

| | 4MMP | released | | | 3MH released | | 3MHA | release |
|---|---|---|---|---|---|---|---|---|
| *Strains* | mean | CV (%) | mean | CV (%) | (*R*)-form (%) | (*S*)-form (%) | mean | CV(%) |
| *WT* | 100 | 5.6 | 100 | 2.7 | 55 | 45 | 100 | |
| *Δure2* | 406.2 ** | 18.6 | 335.1 ** | 19.6 | 77 * | 23* | 231.0 ** | 13.3 |
| *Δirc7* | 5.1** | 0.5 | 62.4 ** | 2.8 | 48 | 52 | 53.3 ** | 1.3 |
| Δ*irc7*Δ*ure2* | 5.0 ** | 0.2 | 64.2 ** | 5.4 | 49 | 51 | 55.2 ** | 5.9 |

| | | | | | | | | |
|---|---|---|---|---|---|---|---|---|
| *Statistically different from *WT* strain, ANOVA P= 0.05. **Statistically different from *WT* strain, ANOVA P= 0.01. | | | | | | | | |

The 4MMP and 3MH release were identical in Δ*irc7* and Δ*ure2* Δ*irc7* strains. Therefore in absence of the Irc7p enzyme, the *URE2* deletion did not increase thiols release. This demonstrated the epispatic relation between *IRC7* and *URE2* suggesting that in derepressed cells, no other enzymes contributing to aroma release were activated through the NCR pathway. Irc7p was thus the sole enzyme whose precursor cleavage activity was transcriptionally induced through NCR regulation pathway. Moreover, when Irc7p was absent, the stoichiometric switch of (*R*)/(*S*)-3MH enantiomers induced by the *URE2* deletion was not observed (Table VI). This finding suggested that the Irc7p enzyme had a best substrate affinity for (*RR*)-P-3MH producing by consequence more (*R*)-3MH. This type of stereoselectivity for the P-3MH diastereoisomers has been previously described for other β-lyases (Wakabayashi et al., J Agric. Food Chem., 2004, 52, 110-116).

### Example 5 : URGE2 mutant yeast strain selection

An *URE2* defective allele was introduced in an industrial strain background (X5 derived strain) by backcross procedure. The *URE2* allele was brought by an EMS mutant obtained from a laboratory yeast strain (Sigma 1219) according to the procedure described in Drillien, R and F. Lacroute (J. Bacteriol., 1972, 109, 203-208). Compared to S288c genome this mutant showed a G182E substitution in the central part of the protein (Figure 2) corresponding to the genomic coordinate 219511 to 219856. This substitution may be tracked by a PCR/RFLP test amplifying this genomic portion by the primers P273 (5'-aaagcttgtccaatcattggc-3', SEQ ID NO : 14) and P274 (5'-ttttctctcacaggtctgcg-3', SEQ ID NO : 15). The mutant allele was detected by cutting the PCR fragment with the enzyme *Mnl*1 that specifically cut said fragment at position 156.

*URE2* mutant allele was followed during backcross experiment as described for other traits by (Marullo et al., 2007 FEMS Yeast Res., 2007, 7, 1295-1306, Epub 2007 Sep 20). After two backcrosses a strain (BC2-3) carrying this allele was compared to the initial parent strain (IP). This strain was expected to share more than 75 % of homology with the initial parent strain. In terms of volatile thiols production this strain produce 3 fold high 3MH that the initial parent strain (Table VI).

**Table VI : Enhancement of 3MH production for a ure2 strain obtained by backcross.**

| Strain | IP | | | BC2-3 | | |
|---|---|---|---|---|---|---|
| Rep* | 1 | 2 | 3 | 1 | 2 | 3 |
| 3MH ng/L | 1040 | 1069 | 798 | 4441 | 4489 | 3536 |

| | | | | | | |
|---|---|---|---|---|---|---|
| * data from triplicate assay | | | | | | |

## Claims

1. Use of a *URE2* mutant yeast having a loss-of-function mutation
in the *URE2* gene for increasing the release of aromatic volatile thiol by yeast during fermentation.

2. The use of claim 1, wherein the mutant is a null mutant lacking Ure2p function.

3. The use of claim 1 or claim 2, wherein the mutant is a mutant of *Saccharomyces sp* or of a derived interspecific hybrid

4. The use of anyone of claims 1 to 3, wherein the mutant is a mutant of a wine yeast strain.

5. The use of claim 4, wherein the wine yeast strain is selected from the group consisting of: VL3c, X5 and RX60.

6. The use of anyone of claims 1 to 5, wherein the aromatic volatile thiol derives from a S-cysteine conjugate precursor.

7. The use of anyone of claims 1 to 6, wherein the volatile thiol is selected from the group consisting of: 4-mercapto-4-methylpentan-2-one (4MMP), 3-mercaptohexan-1-ol (3MH), 4-mercapto-4-methylpentan-2-ol (4MMPOH), 3-mercaptohexyl acetate (3MHA), 3-mercaptopentan-1-ol (3MPOH), 3-mercaptoheptan-1-ol (3MHepOH), 3-mercapto-2-methylbutan-1-ol (3M2MBOH), and/or 3-mercapto-2-methylpentan-1-ol (3M2MPOH).

8. The use of claim 7, wherein the 3MH which is released by the mutant consists of more *(R)* enantiomer than *(S)* enantiomer.

9. The use of claim 8, wherein the 3MH consists of at least 70 % of *(R)* enantiomer.

10. The use of anyone of claims 1 to 9, wherein the release of volatile thiol is increased at least by a factor of 3.

11. The use of anyone of claims 1 to 10, wherein the fermentation is alcoholic fermentation.

12. The use of claim 11, wherein the fermented product is an alcoholic beverage such as wine.

13. The use of claim 12, wherein the wine is made by fermentation of a simple or non-floral grape variety selected from the group consisting of: Sauvignon Blanc, Gewurztraminer, Riesling, Semillon, Colombard, Scheurebe, Petit Manseng, Cabernet Sauvignon and Merlot

14. A method for enhancing the aroma of a fermented product produced by fermentation of an initial product containing at least one non-volatile thiol precursor, comprising the incubation of the initial product with a *URE2* mutant yeast as defined in anyone of claims 1 to 5, under conditions allowing fermentation by the *URE2* mutant to occur, and thereby increase the release of the volatile thiol(s) by the mutant yeast, during fermentation.

15. A method for enhancing the grapefruit overtone of a fermented product produced by fermentation of an initial product containing S-3-(hexan-1-ol)-L-cysteine (3MH precursor), comprising the incubation of the initial product with a *URE2* mutant yeast as defined in anyone of claims 1 to 5, under conditions allowing fermentation by the *URE2* mutant to occur, and thereby increase the release of the 3MH (R) enantiomer, by the mutant yeast, during fermentation.

16. A method for identifying yeasts able to improve the aroma of a fermented product, comprising at least the step of:
(a) obtaining *URE2* mutant yeasts,
(b) inoculating the *URE2* mutant(s) of step (a) in a fermentation medium containing non-volatile thiol precursor(s), under conditions allowing fermentation by the *URE2* mutant(s) to occur, and thereby release of the volatile thiol(s) by the mutant during fermentation,
(c) measuring the volatile thiol(s) released in the fermented medium, by any appropriate means, and
(d) identifying the *URE2* mutant(s) able to release high level volatile thiol, as compared to yeast having a wild-type *URE2* gene in a nearly isogenic genetic background.

## Patentansprüche

1. Verwendung einer *URE2*-Hefemutante mit einer Funktionsverlustmutation im URE2-Gen zum Erhöhen der Freisetzung von aromatischem flüchtigem Thiol durch Hefe während einer Fermentation.

2. Verwendung nach Anspruch 1, wobei die Mutante eine Nullmutante ohne Ure2p-Funktion ist.

3. Verwendung nach Anspruch 1 oder Anspruch 2, wobei die Mutante eine Mutante von *Saccharomyces sp* oder einer abgeleiteten interspezifischen Hybride ist.

4. Verwendung nach einem der Ansprüche 1 bis 3, wobei die Mutante eine Mutante eines Weinhefestamms ist.

5. Verwendung nach Anspruch 4, wobei der Weinhefestamm ausgewählt ist aus der Gruppe bestehend aus: VL3c, X5 und RX60.

6. Verwendung nach einem der Ansprüche 1 bis 5, wobei das aromatische flüchtige Thiol von einem S-Cysteinkonjugat-Vorläufer abgeleitet ist.

7. Verwendung nach einem der Ansprüche 1 bis 6, wobei das flüchtige Thiol ausgewählt ist aus der Gruppe bestehend aus: 4-Mercapto-4-methylpentan-2-on (4MMP), 3-Mercaptohexan-1-ol (3MH), 4-Mercapto-4-methylpentan-2-ol (4MMPOH), 3-Mercaptohexylacetat (3MHA), 3-Mercaptopentan-1-ol (3MPOH), 3-Mercaptoheptan-1-ol (3MHepOH), 3-Mercapto-2-methylbutan-1-ol (3M2MBOH) und/oder 3-Mercapto-2-methylpentan-1-ol (3M2MPOH).

8. Verwendung nach Anspruch 7, wobei das von der Mutante freigesetzte 3MH aus mehr (R)-Enantiomer als (S)-Enantiomer besteht.

9. Verwendung nach Anspruch 8, wobei das 3MH aus wenigstens 70% (R)-Enantiomer besteht.

10. Verwendung nach einem der Ansprüche 1 bis 9, wobei die Freisetzung des flüchtigen Thiols wenigstens um den Faktor 3 erhöht ist.

11. Verwendung nach einem der Ansprüche 1 bis 10, wobei die Fermentation eine alkoholische Fermentation ist.

12. Verwendung nach Anspruch 11, wobei das fermentierte Produkt ein alkoholisches Getränk, wie z.B. Wein, ist.

13. Verwendung nach Anspruch 12, wobei der Wein durch Fermentation einer einfachen oder nichtblühenden Rebsorte hergestellt wird, ausgewählt aus der Gruppe bestehend aus: Sauvignon Blanc, Gewürztraminer, Riesling, Semillon, Colombard, Scheurebe, Petit Manseng, Cabernet Sauvignon und Merlot.

14. Verfahren zum Verbessern des Aromas eines fermentierten Produkts, das durch Fermentation eines Ausgangsprodukts, das wenigstens einen nicht flüchtigen Thiolvorläufer enthält, hergestellt wird, umfassend die Inkubation eines Ausgangsprodukts mit einer *URE2*-Hefemutante nach einem der Ansprüche 1 bis 5, unter Bedingungen, die eine Fermentation durch die *URE2*-Mutante stattfinden lassen, und **dadurch** Erhöhen der Freisetzung des oder der flüchtigen Thiole durch die Hefemutante während der Fermentation.

15. Verfahren zum Erhöhen des Grapefruitobertons eines fermentierten Produkts, das durch Fermentation eines Ausgangsprodukts, das S-3-(Hexan-1-ol)-L-cystein (3MH-Vorläufer) enthält, hergestellt wird, umfassend die Inkubation des Ausgangsprodukts mit einer *URE*2-Mutanten-Hefe nach einem der Ansprüche 1 bis 5, unter Bedingungen, die eine Fermentation durch die *URE2*-Mutante stattfinden lassen, und **dadurch** Erhöhen der Freisetzung des 3MH-(*R*)-Enantiomers durch die Hefemutante während der Fermentation.

16. Verfahren zum Identifizieren von Hefen, die in der Lage sind, das Aroma eines fermentierten Produkts zu erhöhen, umfassend wenigstens den Schritt:
(a) Erhalten von *URE2*-Hefemutanten,
(b) Animpfen der *URE2*-Mutante(n) aus Schritt (a) in einem Fermentationsmedium, das einen oder mehrere nichtflüchtige Thiolvorläufer enthält, unter Bedingungen, die eine Fermentation durch die *URE2*-Mutante(n) stattfinden lassen, und **dadurch** Erhöhen der Freisetzung des oder der flüchtigen Thiole durch die Mutantenhefe während der Fermentation,
(c) Messen mit geeigneten Mitteln des oder der flüchtigen Thiole, die in das fermentierte Medium freigesetzt werden, und
(d) Identifizieren der *URE*2-Mutante(n), die verglichen mit einer Hefe mit dem Wildtyp-*URE2*-Gen in einem annährend isogenen genetischen Hintergrund in der Lage sind, große Mengen flüchtiges Thiol freizusetzen.

## Revendications

**1.** Utilisation d'une levure mutante pour *URE2* ayant une mutation perte de fonction dans le gène *URE2,* pour augmenter la libération de thiol volatil aromatique par la levure pendant la fermentation.

**2.** Utilisation selon la revendication 1, pour laquelle le mutant est un mutant nul, dépourvu de fonction d'Ure2p.

**3.** Utilisation selon la revendication 1 ou 2, pour laquelle le mutant est un mutant de *Saccharomyces sp* ou d'un hybride interspécifique dérivé.

**4.** Utilisation selon l'une quelconque des revendications 1 à 3, pour laquelle le mutant est un mutant d'une souche de levure du vin.

**5.** Utilisation selon la revendication 4, pour laquelle la souche de levure du vin est choisie dans le groupe consistant en VL3c, X5 et RX60.

**6.** Utilisation selon l'une quelconque des revendications 1 à 5, pour laquelle le thiol volatil aromatique dérive d'un précurseur S-conjugué à la cystéine.

**7.** Utilisation selon l'une quelconque des revendications 1 à 6, pour laquelle le thiol volatil est choisi dans le groupe consistant en la 4-mercapto-4-méthylpentan-2-one (4MMP), le 3-mercaptohexan-1-ol (3MH), le 4-mercapto-4-méthylpentan-2-ol (4MMPOH), l'acétate de 3-mercaptohexyle (3MHA), le 3-mercaptopentan-1-ol (3MPOH), le 3-mercaptoheptan-1-ol (3MHepOH), le 3-mercapto-2-méthylbutan-1-ol (3M2MBOH), et/ou le 3-mercapto-2-méthylpentan-1-ol (3M2MPOH).

**8.** Utilisation selon la revendication 7, pour laquelle le 3MH qui est libéré par le mutant consiste en une quantité de l'énantiomère *(R)* plus grande que celle de l'énantiomère *(S).*

**9.** Utilisation selon la revendication 8, pour laquelle le 3MH consiste en au moins 70 % de l'énantiomère *(R).*

**10.** Utilisation selon l'une quelconque des revendications 1 à 9, pour laquelle la libération du thiol volatil est multipliée par un facteur au moins égal à 3.

**11.** Utilisation selon l'une quelconque des revendications 1 à 10, pour laquelle la fermentation est une fermentation alcoolique.

**12.** Utilisation selon la revendication 11, pour laquelle le produit fermenté est une boisson alcoolique telle que le vin.

**13.** Utilisation selon la revendication 12, pour laquelle le vin est obtenu par fermentation d'un cépage simple ou non floral choisi dans le groupe consistant en le Sauvignon blanc, le Gewürztraminer, le Riesling, le Semillon, le Colombard, le Scheurebe, le Petit Manseng, le Cabernet Sauvignon et le Merlot.

**14.** Procédé pour améliorer l'arôme d'un produit fermenté produit par fermentation d'un produit initial contenant au moins un précurseur de thiol non volatil, comprenant l'incubation du produit initial avec une levure mutante pour *URE2* telle que définie dans l'une quelconque des revendications 1 à 5, dans des conditions permettant à la fermentation par le mutant *URE2* de se produire, et de façon à augmenter la libération du ou des thiols volatils par la levure mutante au cours de la fermentation.

**15.** Procédé pour renforcer la touche de pamplemousse d'un produit fermenté produit par fermentation d'un produit initial contenant de la S-3-(hexan-1-ol)-L-cystéine (précurseur de 3MH), comprenant l'incubation du produit initial avec une levure mutante pour *URE2* telle que définie dans l'une quelconque des revendications 1 à 5, dans des conditions permettant à une fermentation par le mutant *URE2* de se produire, de façon à augmenter la libération de l'énantiomère (R) du 3MH, par la levure mutante, pendant la fermentation.

**16.** Procédé pour identifier des levures capables d'améliorer l'arôme d'un produit fermenté, comprenant au moins l'étape
(a) d'obtention de levures mutantes pour *URE2,*
(b) d'inoculation des mutants *URE2* de l'étape (a) dans un milieu de fermentation contenant un ou plusieurs précurseurs de thiol non volatil, dans des conditions permettant à la fermentation par le ou les mutants *URE2* de se produire, de façon à libérer le ou les thiols volatils par le mutant pendant la fermentation,
(c) de mesure, par un moyen approprié quelconque, du ou des thiols volatils libérés dans le milieu fermenté, et
(e) d'identification du ou des mutants *URE2* capables de libérer des quantités importantes du thiol volatil, par comparaison à une levure ayant un gène *URE2* de type sauvage, dans un fond génétique presque isogénique.
